# EUROPEAN PATENT APPLICATION

(11) **EP 4 813 434 A1**
(43) Date of publication of application: **30.09.2026**
(21) Application number: 24912167.4
(22) Date of filing: 27.11.2024
(51) Int. Cl.: A61M 25/09, A61M 25/092

(54) **MEDICAL WIRE**

(30) Priority: 26.12.2023 JP 2023218948
(71) Applicant: NHK Spring Co., Ltd., Yokohama-shi, Kanagawa 236-0004 (JP)
(72) Inventor: IMAMURA, Masaru, Yokohama-shi, Kanagawa 236-0004 (JP); KANAZAWA, Asuka, Yokohama-shi, Kanagawa 236-0004 (JP); ENDO, Takahiro, Yokohama-shi, Kanagawa 236-0004 (JP); HANAYAMA, Yukichi, Yokohama-shi, Kanagawa 236-0004 (JP)
(74) Representative: V.O.
(86) International application number: PCT/JP2024/041918
(87) International publication number: WO 2025/142276

(57) **Abstract**

A medical wire includes a first flexible tube (11) that extends in a front-rear direction and is formed to be bendable, an operation wire (12) that extends in the front-rear direction and is inserted into the first flexible tube (11), and a support body (14) that is fixed to a rear end portion of the first flexible tube (11), in which a front end portion of the operation wire (12) is fixed to a front end portion of the first flexible tube (11) while separated from a central axis (O1) of the first flexible tube (11) in one direction in a radial direction, an operation portion (13) to which the operation wire is fixed and that is movable in the front-rear direction with respect to the support body is provided behind the support body, and a first restricting portion (16) and a second restricting portion (17) that engage with each other to restrict relative movement between the support body and the operation portion in a circumferential direction are formed on the support body and on the operation portion, respectively.

## Description

### TECHNICAL FIELD

The present invention relates to a medical wire such as a guide wire.

Priority is claimed on Japanese Patent Application No. 2023-218948, filed December 26, 2023, the content of which is incorporated herein by reference.

### BACKGROUND ART

In the related art, as a medical wire, a guide wire is known, for example, as shown in Patent Document 1 below, which includes a first flexible tube (outer flexible tube 4) that extends in a front-rear direction and is formed to be bendable, an operation wire (core shaft) that extends in the front-rear direction and is inserted into the first flexible tube, and a support body that is fixed to a rear end portion of the first flexible tube. In general, the guide wire is used to guide a balloon or a stent to a chronic total occlusion lesion part or the like in a blood vessel in a state where the guide wire is inserted into the blood vessel and penetrates the chronic total occlusion lesion part or the like.

In a case where the guide wire penetrates the chronic total occlusion lesion part or the like, for example, the following procedure is performed.

First, a guide wire is made to enter a blood vessel in a state where the guide wire is inserted into a microcatheter, when the guide wire is made to reach a chronic total occlusion lesion part or the like in the blood vessel, the guide wire used so far is pulled out while leaving the microcatheter in the blood vessel, and then a guide wire having a higher distal end load or the like than the guide wire that is pulled out is inserted into the microcatheter and is pierced into the chronic total occlusion lesion part or the like, so that the chronic total occlusion lesion part or the like is penetrated by the guide wire and the microcatheter. Then, the guide wire having a higher distal end load or the like is pulled out while leaving the microcatheter in the blood vessel in a state where the chronic total occlusion lesion part or the like is penetrated by the microcatheter, and the original guide wire having a lower distal end load or the like is inserted into the microcatheter again and is made to reach a position where the chronic total occlusion lesion part or the like is penetrated by the guide wire, and thereafter the microcatheter is pulled out while leaving the guide wire.

### Citation List

### Patent Document

Patent Document 1: Patent Document 1: Japanese Unexamined Patent Application, First Publication No. 2011-199

### SUMMARY OF INVENTION

### Technical Problem

In a guide wire in the related art, to penetrate the chronic total occlusion lesion part of the like, it is necessary to interchangeably use guide wires having different bending stiffnesses such as distal end loads by inserting and removing the guide wires into and from the blood vessel or the like. In addition, to select a blood vessel at a blood vessel bifurcation portion and make the guide wire enter the target blood vessel until making the guide wire reach the chronic total occlusion lesion part or the like, it is necessary to insert and remove the guide wire into and from the blood vessel and finely adjust the curvature (a bending habit called pre-shape) of a front end portion of the guide wire outside the body. There has been a demand for shortening the surgical time.

The present invention provides a medical wire that can shorten the operative time.

### Solution to Problem

A medical wire according to an aspect of the present invention includes a first flexible tube that extends in a front-rear direction and is formed to be bendable, an operation wire that extends in the front-rear direction and is inserted into the first flexible tube, and a support body that is fixed to a rear end portion of the first flexible tube, in which a front end portion of the operation wire is fixed to a front end portion of the first flexible tube while separated from a central axis of the first flexible tube in one direction in a radial direction, an operation portion to which the operation wire is fixed and that is movable in the front-rear direction with respect to the support body is provided behind the support body, and a first restricting portion and a second restricting portion that engage with each other to restrict relative movement between the support body and the operation portion in a circumferential direction are formed on the support body and on the operation portion, respectively.

According to the medical wire according to the aspect of the present invention, the front end portion of the operation wire is fixed to the front end portion of the first flexible tube while separated from the central axis of the first flexible tube in one direction in the radial direction. Therefore, in a case where the operation wire is relatively pulled rearward with respect to the support portion, the first flexible tube is bent in the specific one direction in the radial direction while being compressed and deformed, and it is possible to select a blood vessel at a blood vessel bifurcation portion and make the medical wire enter the target blood vessel while operating the operation wire to bend the front end portion of the medical wire in the specific direction. Accordingly, it is possible to reduce the dependence on the operation feeling of the doctor in a case where the medical wire is made to reach a chronic total occlusion lesion part or the like while advanced along the blood vessel, and it is possible to eliminate a need for inserting and removing the medical wire into and from the blood vessel to finely adjust the curvature of the front end portion of the medical wire outside the body.

Further, in a case where the operation wire is relatively pulled rearward with respect to the support portion, the front end portion of the operation wire and the front end portion of the first flexible tube move rearward, and the first flexible tube is compressed and deformed in the front-rear direction between the first flexible tube and the support body, so that bending stiffness of the first flexible tube is increased. Therefore, the bending stiffness of the first flexible tube can be changed by operating the operation wire in a state where the medical wire is inserted into the blood vessel.

As a result, in a case where the medical wire penetrates the chronic total occlusion lesion part or the like, the bending stiffness of the first flexible tube is lowered until the medical wire enters the blood vessel and reaches the chronic total occlusion lesion part or the like in the blood vessel, the bending stiffness of the first flexible tube is increased by relatively pulling the operation wire rearward with respect to the support portion when the medical wire is pierced into the chronic total occlusion lesion part or the like and the medical wire penetrates the chronic total occlusion lesion part or the like, and after the penetration of the chronic total occlusion lesion part or the like, the operation of the operation wire is released to return the bending stiffness of the first flexible tube to an original low state. Therefore, it is possible to eliminate a need to interchangeably use medical wires having different bending stiffnesses by inserting and removing the medical wires into and from the blood vessel or the like in order to penetrate the chronic total occlusion lesion part or the like.

As described above, it is possible to shorten the operative time.

Since the operation portion to which the operation wire is fixed is provided behind the support body, it is possible to improve the operability of the medical wire as compared with a case where the operation wire is directly operated.

The first restricting portion and the second restricting portion that engage with each other to restrict the relative movement between the support body and the operation portion in the circumferential direction are formed on the support body and on the operation portion, respectively. Therefore, in a case where the operation portion is operated, it is possible to restrict the operation portion from rotationally moving with respect to the support body, and it is possible to reliably improve the operability of the medical wire and to easily assemble the operation portion to the support body.

One of the first restricting portion and the second restricting portion may be a protrusion, the other of the first restricting portion and the second restricting portion may be a recessed portion in which the protrusion is accommodated to be movable in the front-rear direction, and the recessed portion may be provided with an abutting portion that abuts against the protrusion in the front-rear direction in a case where the operation portion moves rearward with respect to the support body.

Since the recessed portion is provided with the abutting portion that abuts against the protrusion in the front-rear direction in a case where the operation portion moves rearward with respect to the support body, the rearward movement amount of the operation wire is controlled to fall within a desired range, and it is possible to prevent an excessively large load from being applied to the first flexible tube.

A second flexible tube that extends in the front-rear direction and is formed to be bendable may be inserted into the first flexible tube, the operation wire may be inserted into the second flexible tube, a central axis of the second flexible tube and the front end portion of the operation wire may be separated from a central axis of the first flexible tube in the one direction in the radial direction, a front end portion of the second flexible tube may be fixed to at least one of the front end portion of the first flexible tube and the front end portion of the operation wire, and a rear part of the second flexible tube located rearward of the front end portion may be fixed to at least one of the support body and the rear end portion of the first flexible tube.

Since the second flexible tube is provided, in a case where the operation wire is relatively pulled rearward with respect to the support portion, the first flexible tube and the second flexible tube are bent in one direction in the radial direction while being compressed and deformed. Therefore, for example, it is possible to easily adjust (design) the relationship between the operation force on the operation wire and the bending shape of the front end portion of the medical wire while maintaining the outer diameter of the medical wire.

### Advantageous Effects of Invention

According to the above-described aspects of the present invention, it is possible to shorten the operative time.

### BRIEF DESCRIPTION OF DRAWINGS

[FIG. 1] A longitudinal cross-sectional view of a medical wire of a first embodiment.
[FIG. 2A] A cross-sectional view taken along line II-II of FIG. 1.
[FIG. 2B] A first modification example of the medical wire according to the embodiment.
[FIG. 2C] A second modification example of the medical wire according to the embodiment.
[FIG. 2D] A third modification example of the medical wire according to the embodiment.

### DESCRIPTION OF EMBODIMENTS

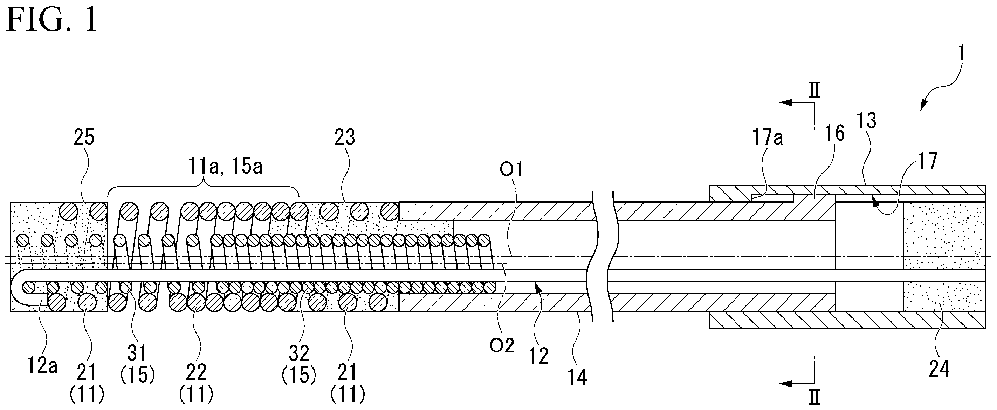

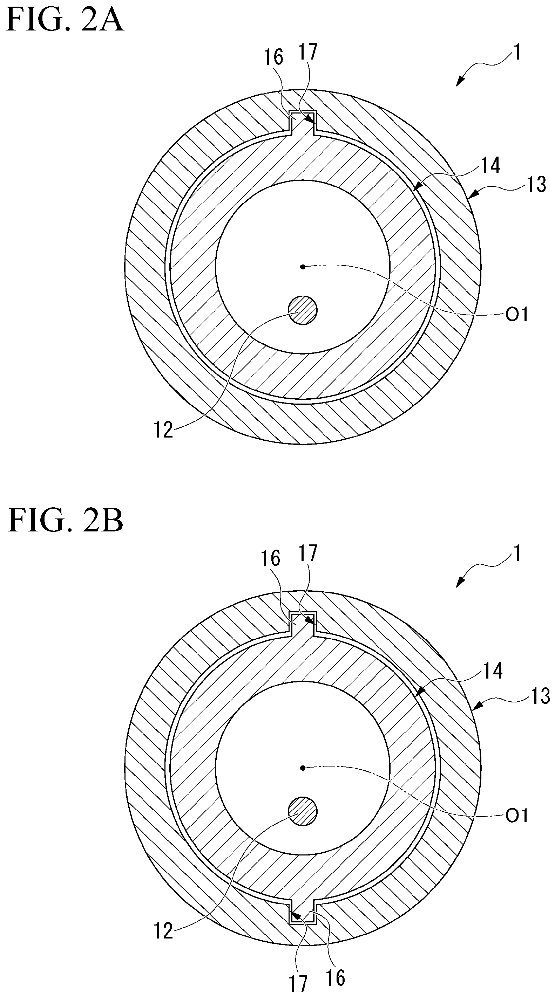

Hereinafter, a first embodiment of a medical wire will be described with reference to FIGS. 1 and 2A. A medical wire 1 is a guide wire including a first flexible tube 11, an operation wire 12, an operation portion 13, a support body 14, and a second flexible tube 15. The first flexible tube 11, the operation wire 12, the operation portion 13, the support body 14, and the second flexible tube 15 are formed of, for example, a metal material or the like. A material for forming the first flexible tube 11, the operation wire 12, the operation portion 13, the support body 14, and the second flexible tube 15 may be changed as appropriate. The second flexible tube 15 may not be provided.

The operation portion 13 and the support body 14 are formed in a tubular shape and are disposed coaxially with a central axis O1 of the first flexible tube 11.

Hereinafter, a side on which the first flexible tube 11 is located with respect to the support body 14 in a direction in which the central axis O1 extends is referred to as a front side, a side on which the operation portion 13 is located with respect to the support body 14 in the direction in which the central axis O1 extends is referred to as a rear side, and a direction in which the central axis O1 extends is referred to as a front-rear direction. A direction intersecting the central axis O1 when viewed in the front-rear direction will be referred to as a radial direction, and a direction that extends circumferentially around the central axis O1 when viewed in the front-rear direction will be referred to as a circumferential direction.

The first flexible tube 11 extends in the front-rear direction and is formed to be bendable. The first flexible tube 11 is a coil spring extending in the front-rear direction.

The outer diameter of the first flexible tube 11 is a size that allows the first flexible tube 11 to be inserted into a blood vessel (for example, 0.2 mm or more and 1.0 mm or less). The outer shape and the size of the first flexible tube 11 as viewed in the front-rear direction are the same over the entire length in the front-rear direction. The diameter of a wire material forming the first flexible tube 11 is the same over the entire length.

The diameter of the wire material forming the first flexible tube 11 may not be the same over the entire length, and the outer shape and the size of the first flexible tube 11 as viewed in the front-rear direction may not be the same over the entire length in the front-rear direction. For example, the outer diameter of the first flexible tube 11 may decrease toward the front side. The first flexible tube **11** is not limited to the coil spring, and may be, for example, a tube having a peripheral wall that continuously extends over the entire length in each of the front-rear direction and the circumferential direction.

The support body 14 is fixed to a rear end portion of the first flexible tube 11. The support body 14 extends in the front-rear direction and is formed to be bendable. A rear end opening edge of the first flexible tube 11 abuts on a front end opening edge of the support body 14 in the front-rear direction. A front end portion of the support body 14 and the rear end portion of the first flexible tube **11** are fixed to each other by, for example, soldering, brazing, adhesion, or crimping.

In the illustrated example, for example, a tubular intermediate fixing member 23 including a solder material, a brazing material, an adhesive, or the like extends forward from the front end portion of the support body 14. An outer peripheral surface of the intermediate fixing member 23 is connected to outer peripheral surfaces of the support body 14 and the first flexible tube 11 in the front-rear direction without a step.

The outer diameter of the support body 14 is the same as the outer diameter of the first flexible tube 11 and has a size that allows the support body 14 to be inserted into a blood vessel (for example, 0.2 mm or more and 1.0 mm or less). The outer shape and the size of the support body 14 when viewed in the front-rear direction are the same over the entire length in the front-rear direction.

The outer shape and the size of the support body 14 when viewed in the front-rear direction may not be the same over the entire length in the front-rear direction. For example, the outer diameter of the support body 14 may decrease toward the front side. The outer shapes and the sizes of the support body 14 and the first flexible tube 11 when viewed in the front-rear direction may be different from each other. In this case, it is preferable that the support body 14 does not protrude outward in the radial direction from the outer peripheral surface of the first flexible tube 11 when viewed in the front-rear direction.

The operation portion 13 is provided behind the support body 14. The operation portion 13 protrudes rearward from the support body 14. The operation portion 13 is provided to be movable in the front-rear direction with respect to the support body 14. In the illustrated example, a rear end portion of the support body 14 is inserted into a front part of the operation portion 13. A front end opening edge of the operation portion 13 and a rear end opening edge of the support body 14 may face each other in the front-rear direction, or the front part of the operation portion 13 may be inserted into the rear end portion of the support body 14.

A rear end portion of the operation wire 12 is fixed to the operation portion 13 by, for example, soldering, brazing, adhesion, or crimping. In the illustrated example, a rear end opening portion of the operation portion 13 is closed by, for example, a rear fixing member 24 including a solder material, a brazing material, an adhesive, or the like, and the rear end portion of the operation wire 12 is embedded in the rear fixing member 24.

The operation wire 12 extends in the front-rear direction and is inserted into the first flexible tube 11. The operation wire 12 is formed to be elastically deformable. A front end portion of the operation wire 12 is fixed to a front end portion of the first flexible tube 11 by, for example, soldering, brazing, adhesion, or crimping while separated from the central axis O1 in one direction in the radial direction.

A plurality of the operation wires 12 may be provided at intervals in the circumferential direction.

The operation wire 12 extends straight in the front-rear direction. The operation wire 12 may extend in the front-rear direction while bent, for example. A rear part of the operation wire 12 protrudes rearward from the support body 14. A protruding portion 12a of the front end portion of the operation wire 12, which protrudes forward from the first flexible tube 11, is bent in the one direction in the radial direction and is locked to a front end opening edge of the first flexible tube 11. The protruding portion 12a of the operation wire 12 is located at the same radial position as, or radially inward from, the outer peripheral surface of the front end portion of the first flexible tube 11. The protruding portion 12a of the operation wire 12 is bent in the one direction in the radial direction and is folded rearward, and a distal end surface of the operation wire 12 faces rearward. The distal end surface of the operation wire 12 abuts on or is close to the front end opening edge of the first flexible tube 11. The distal end surface of the operation wire 12 may face the one direction in the radial direction.

In the illustrated example, a front end opening portion of the first flexible tube 11 is closed by, for example, a front fixing member 25 including a solder material, a brazing material, an adhesive, or the like. An outer peripheral surface of the front fixing member 25 is connected to the outer peripheral surface of the first flexible tube 11 without a step in the front-rear direction. The front end portion of the operation wire 12 including the protruding portion 12a is embedded in the front fixing member 25.

In the first flexible tube 11, a bending stiffness of an intermediate portion 11a located between the front end portion (front fixing member 25) and the rear end portion (intermediate fixing member 23) decreases from the rear side toward the front side. In the illustrated example, the first flexible tube 11 includes a first rigid tube portion 22 having high bending stiffness, and a pair of first flexible tube portions 21 which have lower bending stiffness than the first rigid tube portion 22 and between which the first rigid tube portion 22 is interposed in the front-rear direction. Of the pair of first flexible tube portions 21, the number of turns of the first flexible tube portion 21 located on the front side is larger than the number of turns of the first flexible tube portion 21 located on the rear side. The number of turns of the former may be equal to or less than the number of turns of the latter.

The first flexible tube portion 21 is formed such that an interval (wire material gap) between wire materials adjacent to each other in the front-rear direction is larger than that of the first rigid tube portion 22. As a result, the bending stiffness of the first flexible tube portion 21 is smaller than the bending stiffness of the first rigid tube portion 22, and the first flexible tube portion 21 is easily flexibly bent following the bent blood vessel.

In the illustrated example, the wire materials adjacent to each other in the front-rear direction in the first rigid tube portion 22 abut on each other. That is, the first rigid tube portion 22 is a tightly wound coil spring. As a result, in the intermediate portion 11a of the first flexible tube 11, the first rigid tube portion 22 is easily flexibly bent following the bending deformation of the first flexible tube portion 21.

A front part of the first flexible tube portion 21 located on the front side of the pair of first flexible tube portions 21 is joined to or adhered to the front fixing member 25, and a rear part of the first flexible tube portion 21 located on the front side is located between the front fixing member 25 and the intermediate fixing member 23. The first flexible tube portion 21 located on the rear side of the pair of first flexible tube portions 21 is joined to or adhered to the intermediate fixing member 23 over the entire region.

Therefore, in the first flexible tube 11, the intermediate portion 11a located between the front fixing member 25 and the intermediate fixing member 23 is composed of the entire first rigid tube portion 22 and the rear part of the first flexible tube portion 21 located on the front side of the pair of first flexible tube portions 21. As a result, the bending stiffness of the intermediate portion 11a of the first flexible tube 11 decreases stepwise from the rear side toward the front side. The bending stiffness of the intermediate portion 11a of the first flexible tube 11 may linearly decrease from the rear side toward the front side.

In the illustrated example, the length of the first rigid tube portion 22 in the front-rear direction is longer than the length of the rear part of the first flexible tube portion 21 located on the front side of the pair of first flexible tube portions 21 in the front-rear direction. The length of the former may be equal to or less than the length of the latter.

The second flexible tube 15 extends in the front-rear direction and is formed to be bendable. The second flexible tube 15 is inserted into the first flexible tube 11. A front end portion of the second flexible tube 15 is fixed to at least one of the front end portion of the first flexible tube 11 and the front end portion of the operation wire 12, and a rear part of the second flexible tube 15 located rearward of the front end portion is fixed to at least one of the support body 14 and the rear end portion of the first flexible tube 11.

The second flexible tube 15 protrudes from the first flexible tube 11 both forward and backward in the front-rear direction. The second flexible tube 15 may not protrude from the first flexible tube 11 in the front-rear direction.

At least one of the first flexible tube 11 and the second flexible tube 15 is a coil spring extending in the front-rear direction. In the illustrated example, both the first flexible tube 11 and the second flexible tube 15 are coil springs extending in the front-rear direction. Winding directions of the first flexible tube 11 and the second flexible tube 15 are opposite to each other.

The winding directions of the first flexible tube 11 and the second flexible tube 15 may be the same as each other. Neither of the first flexible tube 11 and the second flexible tube 15 is limited to the coil spring, and either may be, for example, a tube having a peripheral wall that continuously extends over the entire length in each of the front-rear direction and the circumferential direction.

In the second flexible tube 15, the bending stiffness of an intermediate portion 15a located between the front end portion (front fixing member 25) and the rear part (intermediate fixing member 23) gradually decreases from the rear side toward the front side. In the illustrated example, the second flexible tube 15 is composed of a second flexible tube portion 31 having low bending stiffness and a second rigid tube portion 32 having higher bending stiffness than the second flexible tube portion 31, which are arranged in that order from the front side toward the rear side. The second flexible tube portion 31 protrudes forward from the first flexible tube 11, and the second rigid tube portion 32 protrudes rearward from the first flexible tube 11. The length of the second flexible tube portion 31 in the front-rear direction is slightly shorter than the length of the second rigid tube portion 32 in the front-rear direction.

The second flexible tube portion 31 is formed such that an interval (wire material gap) between wire materials adjacent to each other in the front-rear direction is larger than that of the second rigid tube portion 32. As a result, the bending stiffness of the second flexible tube portion 31 is smaller than the bending stiffness of the second rigid tube portion 32, and the second flexible tube portion 31 is easily flexibly bent following the bent blood vessel.

In the illustrated example, the wire materials adjacent to each other in the front-rear direction in the second rigid tube portion 32 abut on each other. That is, the second rigid tube portion 32 is a tightly wound coil spring. As a result, in the intermediate portion 15a of the second flexible tube 15, the second rigid tube portion 32 is easily flexibly bent following the bending deformation of the second flexible tube portion 31.

A front part of the second flexible tube portion 31 is joined or adhered to the front fixing member 25. As a result, the front end portion of the second flexible tube 15 is fixed to the front end portion of the first flexible tube 11 and the front end portion of the operation wire 12 via the front fixing member 25. A rear part of the second flexible tube portion 31 is located between the front fixing member 25 and the intermediate fixing member 23.

The intermediate fixing member 23 is joined or adhered to an outer peripheral surface of a portion of the second rigid tube portion 32 which is located between a front end portion located in the first flexible tube 11 and a rear end portion located in the support body 14. As a result, the rear part of the second flexible tube 15 located rearward of the front end portion is fixed to the support body 14 and the rear end portion of the first flexible tube 11 via the intermediate fixing member 23. In the illustrated example, the intermediate fixing member 23 does not close the inside of the second rigid tube portion 32, and the inside of the support body 14 communicates with the inside of the rear part of the second flexible tube portion 31 through the inside of the second rigid tube portion 32.

The intermediate portion 15a of the second flexible tube 15 located between the front fixing member 25 and the intermediate fixing member 23 is composed of the rear part of the second flexible tube portion 31 and the front end portion of the second rigid tube portion 32. As a result, the bending stiffness of the intermediate portion 15a of the second flexible tube 15 decreases stepwise from the rear side toward the front side. The bending stiffness of the intermediate portion 15a of the second flexible tube 15 may linearly decrease from the rear side toward the front side.

In the illustrated example, the length of the rear part of the second flexible tube portion 31 in the front-rear direction is longer than the length of the front end portion of the second rigid tube portion 32 in the front-rear direction. The length of the former may be equal to or less than the length of the latter. The rear part of the second flexible tube portion 31 located in the intermediate portion 11a of the first flexible tube 11 straddles, in the front-rear direction, a boundary portion between the first rigid tube portion 22 and the first flexible tube portion 21 located on the front side of the pair of first flexible tube portions 21.

The operation wire 12 is inserted into the second flexible tube 15. A central axis O2 of the second flexible tube 15 and the front end portion of the operation wire 12 are separated from the central axis O1 of the first flexible tube 11 in the one direction in the radial direction.

The front end portion of the operation wire 12 is fixed to the front end portion of the second flexible tube 15 by, for example, soldering, brazing, adhering, or crimping while separated from the central axis O2 of the second flexible tube 15 in the one direction in the radial direction. The operation wire 12 is disposed on an inner peripheral surface of the second flexible tube 15.

The protruding portion 12a of the operation wire 12 protrudes forward from the second flexible tube 15, is bent in the one direction in the radial direction, and is locked to a front end opening edge of the second flexible tube 15. The protruding portion 12a of the operation wire 12 is folded rearward, and a part of the front end portion of the second flexible tube 15 in a circumferential direction is sandwiched in the radial direction by the front end portion of the operation wire 12 including the protruding portion 12a. The entire front end portion of the operation wire 12 including the protruding portion 12a is embedded in the front fixing member 25.

In the medical wire 1, the outer peripheral surface of the front part of the support body 14 and the outer peripheral surface of a portion located forward of the front part of the support body 14 are covered with a hydrophilic material. As a result, the medical wire 1 can be smoothly moved in the blood vessel with less catching.

In the medical wire 1, the outer peripheral surface of a portion located rearward of the front part of the support body 14 is covered with a hydrophobic material. As a result, it is possible to make it difficult for a hand operating the medical wire 1 to slip. The outer peripheral surface of the operation portion 13 may not be covered with the hydrophobic material.

Examples of the hydrophilic material include polyvinylpyrrolidone, a maleic acid-based resin, and a hyaluronic acid-based resin, and examples of the hydrophobic material include a fluorine-based resin (PTFE, PFA, and the like) and a silicone-based resin.

In the present embodiment, a first restricting portion 16 and a second restricting portion 17 that engage with each other to restrict relative movement between the support body 14 and the operation portion 13 in the circumferential direction are formed on the support body 14 and on the operation portion 13, respectively. One of the first restricting portion 16 and the second restricting portion 17 is a protrusion, and the other of the first restricting portion 16 and the second restricting portion 17 is a recessed portion in which the protrusion is accommodated to be movable in the front-rear direction.

In the illustrated example, the first restricting portion 16 of the support body 14 is a protrusion protruding outward in the radial direction from the outer peripheral surface of the support body 14, and the second restricting portion 17 of the operation portion 13 is a recessed portion recessed outward in the radial direction from an inner peripheral surface of the operation portion 13. One first restricting portion 16 and one second restricting portion 17 are provided.

The first restricting portion 16 is formed in a rectangular parallelepiped shape extending in the front-rear direction. The first restricting portion 16 may be formed in a rod shape or the like extending in the radial direction. As shown in FIG. 2A, the first restricting portion 16 has a quadrangular shape in a transverse cross-sectional view in the radial direction and orthogonal to the front-rear direction, and in two pairs of sides defining the quadrangular shape, one pair of sides rises from the outer peripheral surface of the support body 14, and the other pair of sides extends in the circumferential direction. The size (width) of the first restricting portion 16 in the circumferential direction is the same over the entire region in the radial direction. A rear end edge of the first restricting portion 16 is connected to the rear end opening edge of the support body 14 without a step in the radial direction.

The second restricting portion 17 is a groove that is not through in the radial direction and extends in the front-rear direction. The second restricting portion 17 is open to a rear end opening edge of the operation portion 13 and is located behind the front end opening edge of the operation portion 13. The size (width) of the second restricting portion 17 in the circumferential direction is the same over the entire region in the radial direction.

In an inner surface defining the second restricting portion 17, a pair of side surfaces facing each other in the circumferential direction abut on or are close to the first restricting portion 16 in the circumferential direction. In the inner surface of the second restricting portion 17, a front end surface located at the front end and facing the rear side is separated forward from the first restricting portion 16 in a standby state in which a load is not applied to the first flexible tube 11 and the second flexible tube 15 from the operation wire 12. The front end surface of the second restricting portion 17 is an abutting portion 17a that abuts against the first restricting portion 16 in the front-rear direction in a case where the operation portion 13 moves rearward with respect to the support body 14.

As described above, with the medical wire 1 according to the present embodiment, the front end portion of the operation wire 12 is fixed to the front end portion of the first flexible tube **11** while separated from the central axis O1 of the first flexible tube **11** in one direction in the radial direction. Therefore, in a case where the operation wire 12 is relatively pulled rearward with respect to the support portion 14, the first flexible tube **11** is bent in the one direction in the radial direction while being compressed and deformed, and it is possible to select a blood vessel at a blood vessel bifurcation portion and make the medical wire 1 enter the target blood vessel while operating the operation wire 12 to bend the front end portion of the medical wire 1 in a desired direction. Accordingly, it is possible to reduce dependence on the operation feeling of the doctor in a case where the medical wire 1 is made to reach a chronic total occlusion lesion part or the like while advanced along the blood vessel, and it is possible to eliminate a need for inserting and removing the medical wire into and from the blood vessel to finely adjust the curvature of the front end portion of the medical wire outside the body.

Further, in a case where the operation wire 12 is relatively fully pulled rearward with respect to the support portion 14, the front end portion of the operation wire 12 and the front end portion of the first flexible tube 11 move rearward, and the first flexible tube 11 is compressed and deformed in the front-rear direction between the first flexible tube 11 and the support body 14, so that the bending stiffness of the first flexible tube 11 is increased. Therefore, the bending stiffness of the first flexible tube 11 can be changed by operating the operation wire 12 in a state where the medical wire 1 is inserted into the blood vessel.

As a result, in a case where the medical wire 1 penetrates a chronic total occlusion lesion part or the like, the bending stiffness of the first flexible tube 11 is lowered until the medical wire 1 enters the blood vessel and reaches the chronic total occlusion lesion part or the like in the blood vessel, the bending stiffness of the first flexible tube 11 is increased by relatively fully pulling the operation wire 12 rearward with respect to the support portion 14 when the medical wire 1 is pierced into the chronic total occlusion lesion part or the like and the medical wire 1 penetrates the chronic total occlusion lesion part or the like, and after the penetration of the chronic total occlusion lesion part or the like, the operation of the operation wire 12 is released to return the bending stiffness of the first flexible tube 11 to the original low state. Therefore, it is possible to eliminate a need to interchangeably use medical wires having different bending stiffnesses by inserting and removing the medical wires into and from the blood vessel or the like in order to penetrate the chronic total occlusion lesion part or the like.

As described above, it is possible to shorten the operative time.

Since the operation portion 13 to which the operation wire 12 is fixed is provided behind the support body 14, it is possible to improve the operability of the medical wire 1 as compared with a case where the operation wire 12 is directly operated.

The first restricting portion 16 and the second restricting portion 17 that engage with each other to restrict relative movement between the support body 14 and the operation portion 13 in the circumferential direction are formed on the support body 14 and on the operation portion 13, respectively. Therefore, in a case where the operation portion 13 is operated, it is possible to restrict the operation portion 13 from rotationally moving with respect to the support body 14, and it is possible to reliably improve the operability of the medical wire 1 and to easily assemble the operation portion 13 to the support body 14.

One of the first restricting portion 16 and the second restricting portion 17 is a protrusion, and the other of the first restricting portion 16 and the second restricting portion 17 is a recessed portion in which the protrusion is accommodated to be movable in the front-rear direction, and the recessed portion is provided with the abutting portion 17a that abuts against the protrusion in the front-rear direction in a case where the operation portion 13 moves rearward with respect to the support body 14. Therefore, the rearward movement amount of the operation wire 12 is controlled to fall within a desired range, and it is possible to prevent an excessively large load from being applied to the first flexible tube 11.

Since the second flexible tube 15 is provided, in a case where the operation wire 12 is relatively pulled rearward with respect to the support portion 14, the first flexible tube **11** and the second flexible tube 15 are bent in the one direction in the radial direction while being compressed and deformed. Therefore, for example, it is possible to easily adjust (design) the relationship between the operation force on the operation wire 12 and the bending shape of the front end portion of the medical wire 1 while maintaining the outer diameter of the medical wire 1.

The technical scope of the present invention is not limited to the above embodiment, and various modifications can be made without departing from the spirit of the present invention.

For example, the bending stiffnesses of the intermediate portions 11a and 15a may be the same over the entire length in the front-rear direction.

The protruding portion 12a of the operation wire 12 may be bent in the one direction in the radial direction such that the distal end surface of the operation wire 12 faces the one direction in the radial direction and the protruding portion 12a faces the front end opening edge of the first flexible tube 11 in the front-rear direction while separated forward with respect to the front end opening edge of the first flexible tube 11. In this case, the front fixing member 25 may be filled between the protruding portion 12a of the operation wire 12 and the front end opening edge of the first flexible tube 11, and the front end portion of the operation wire 12 including the protruding portion 12a may be embedded in the front fixing member 25.

The second flexible tube 15 may not be provided, and the protruding portion 12a of the operation wire 12 may be bent in the one direction in the radial direction and locked to the front end opening edge of the first flexible tube 11.

A plurality of the first restricting portions 16 and a plurality of the second restricting portions 17 may be provided, and for example, as shown in FIG. 2B, one first restricting portion 16 and one second restricting portion 17 may be provided for each of both sides of the central axis O1 in the radial direction with the central axis O1 interposed therebetween.

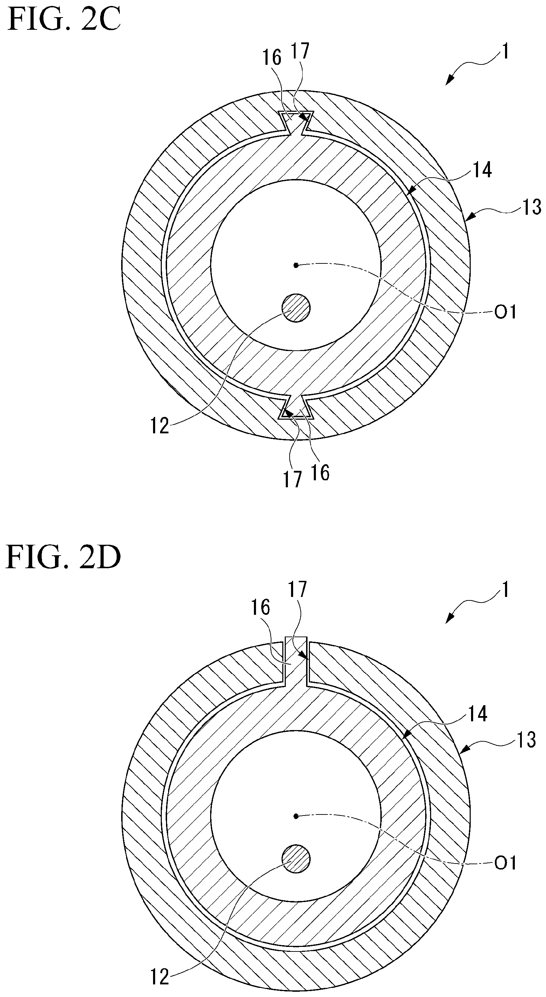

A configuration in which the circumferential sizes of the first restricting portion 16 and the second restricting portion 17 increase toward the outer side in the radial direction may be adopted. For example, as shown in FIG. 2C, the pair of side surfaces of the inner surface of the second restricting portion 17 facing each other in the circumferential direction may extend in a direction in which the side surfaces are separated from each other in the circumferential direction toward the outer side in the radial direction, and the surfaces of the first restricting portion 16 that abut on or are close to the side surfaces may also extend in a direction in which the surfaces are separated from each other in the circumferential direction toward the outer side in the radial direction.

As shown in FIG. 2D, a configuration in which the second restricting portion 17 penetrates the operation portion 13 in the radial direction may be adopted, and a configuration in which the first restricting portion 16 passes through the second restricting portion 17 and protrudes from the outer peripheral surface of the operation portion 13 toward the outer side in the radial direction may be adopted.

The outer end surface of the first restricting portion 16 in the radial direction may be formed in a curved shape protruding toward the outer side in the radial direction or may be recessed toward the inner side in the radial direction, or the first restricting portion 16 may be pointed toward the outer side in the radial direction.

The first restricting portion 16 and the second restricting portion 17 may extend in an inclined direction with respect to the front-rear direction.

As the first restricting portion 16 and the second restricting portion 17, the outer peripheral surface of the support body 14 and the inner peripheral surface of the operation portion 13, which have a non-circular shape in a cross-sectional view in the radial direction and orthogonal to the front-rear direction, may be adopted. In other words, the outer peripheral surface of the support body 14 and the inner peripheral surface of the operation portion 13 may have non-circular shapes similar to each other in the cross-sectional view, the outer peripheral surface of the support body 14 may be configured as the first restricting portion 16, and the inner peripheral surface of the operation portion 13 may be configured as the second restricting portion 17. The first restricting portion 16 and the second restricting portion 17 may be formed by performing plastic working on the support body 14 and the operation portion 13.

As the first restricting portion 16 of the support body 14, a recessed portion recessed from the outer peripheral surface of the support body 14 toward the inner side in the radial direction may be adopted, and as the second restricting portion 17 of the operation portion 13, a protrusion protruding from the inner peripheral surface of the operation portion 13 toward the inner side in the radial direction may be adopted. In addition, the protrusion may be formed by pressing the outer peripheral surface of the operation portion 13 toward the inner side in the radial direction to plastically deform the operation portion 13.

In addition, it is possible to appropriately replace the constituent elements in the above embodiment with well-known constituent elements without departing from the spirit of the present invention. Additionally, the above-described embodiment and the modification examples may be combined with each other as appropriate.

### REFERENCE SIGNS LIST

1: medical wire
11: first flexible tube
12: operation wire
13: operation portion
14: support body
15: second flexible tube
16: first restricting portion
17: second restricting portion
17a: abutting portion
O1: central axis of first flexible tube
O2: central axis of second flexible tube

## Claims

1. A medical wire comprising:
a first flexible tube that extends in a front-rear direction and is formed to be bendable;
an operation wire that extends in the front-rear direction and is inserted into the first flexible tube; and
a support body that is fixed to a rear end portion of the first flexible tube,
wherein a front end portion of the operation wire is fixed to a front end portion of the first flexible tube while separated from a central axis of the first flexible tube in one direction in a radial direction,
an operation portion to which the operation wire is fixed and that is movable in the front-rear direction with respect to the support body is provided behind the support body, and
a first restricting portion and a second restricting portion that engage with each other to restrict relative movement between the support body and the operation portion in a circumferential direction are formed on the support body and on the operation portion, respectively.

2. The medical wire according to claim 1,
wherein one of the first restricting portion and the second restricting portion is a protrusion, and the other of the first restricting portion and the second restricting portion is a recessed portion in which the protrusion is accommodated to be movable in the front-rear direction, and
the recessed portion is provided with an abutting portion that abuts against the protrusion in the front-rear direction in a case where the operation portion moves rearward with respect to the support body.

3. The medical wire according to claim 1 or 2,
wherein a second flexible tube that extends in the front-rear direction and is formed to be bendable is inserted into the first flexible tube,
the operation wire is inserted into the second flexible tube,
a central axis of the second flexible tube and the front end portion of the operation wire are separated from the central axis of the first flexible tube in the one direction in the radial direction,
a front end portion of the second flexible tube is fixed to at least one of the front end portion of the first flexible tube and the front end portion of the operation wire, and
a rear part of the second flexible tube located rearward of the front end portion is fixed to at least one of the support body and the rear end portion of the first flexible tube.
